# EUROPEAN PATENT SPECIFICATION

(11) **EP 3 981 423 B1**
(45) Date of publication and mention of the grant of the patent: **01.05.2024**
(21) Application number: 20857571.2
(22) Date of filing: 17.07.2020
(51) Int. Cl.: A61K 38/47, A61P 19/06, A61P 13/12, C12N 9/36

(54) **COMPOSITION FOR PREVENTING OR TREATING URIC ACID-RELATED DISEASE**
ZUSAMMENSETZUNG ZUR VORBEUGUNG ODER BEHANDLUNG VON KRANKHEITEN IN ZUSAMMENHANG MIT HARNSÄURE
COMPOSITION POUR LA PRÉVENTION OU LE TRAITEMENT D'UNE MALADIE LIÉE À L'ACIDE URIQUE

(30) Priority: 29.08.2019 CN 201910809138
(43) Date of publication of application: 13.04.2022
(73) Proprietor: Guangzhou Century Clinical Research Co., Ltd, Guangzhou, Guangdong 510530 (CN); Guangzhou Xin-Chuangyi Biopharmaceutical Co., Ltd, Huangpu District Guangzhou, Guangdong 510535 (CN); Guangzhou Welman New Drug R&D Co,. Ltd., Guangzhou, Guangdong 510620 (CN); Xiangbei Welman Pharmaceutical Co., Ltd, Hunan 410331 (CN); Nanjing Kangfushun Pharmaceutical Co., Ltd, Nanjing, Jiangsu 210046 (CN)
(72) Inventor: SUN, Mingjie, Guangzhou, Guangdong 510620 (CN); SUN, Tianyu, Guangzhou, Guangdong 510620 (CN); JIANG, Juanyan, Guangzhou, Guangdong 510620 (CN)
(74) Representative: Kraus & Lederer PartGmbB
(86) International application number: PCT/CN2020/102823
(87) International publication number: WO 2021/036572

(56) References cited:
- WO-A1-2018/127532
- WO-A1-2018/127532
- CN-A- 103 316 333
- CN-A- 103 316 333
- CN-A- 105 688 197
- CN-A- 105 688 197
- CN-A- 107 899 006
- CN-A- 107 899 006
- JP-A- 2016 056 199
- JP-A- 2016 056 199
- KR-B1- 101 656 726
- KR-B1- 101 656 726
- US-A1- 2017 246 262
- US-A1- 2017 246 262

## Description

### TECHNICAL FIELD

The present disclosure relates to the field of medicine, in particular to lysozyme for use in the prevention or treatment of a disease related to an excessively high blood uric acid level.

### BACKGROUND

Uric acid is a metabolite in organisms. The precursors thereof are purine substances, which can be divided into endogenous and exogenous. Endogenous substances include nucleic acids and other purine compounds (such as adenine and guanine) that are decomposed by cell metabolism, and exogenous substances include purines ingested from food. Hypoxanthine and xanthine are direct precursors of uric acid. Under the action of xanthine oxidase, hypoxanthine is oxidized into xanthine, and xanthine is oxidized into uric acid.

Since the human body lacks an enzyme (uric acid oxidase) that further metabolizes uric acid, uric acid is excreted as a final product. About two-thirds of uric acid is excreted through the kidneys via urine, and about one-third of uric acid is excreted through the intestinal tract via feces.

The normal uric acid level in human blood is less than 420 µmol/L for male and less than 360 µmol/L for female. When disorders of purine metabolism or uric acid excretion occur *in vivo,* the blood uric acid level often exceeds the normal range, presenting pathological conditions such as hyperuricemia. Persistent high uric acid may also cause other metabolic diseases, cardiovascular and cerebrovascular diseases, inflammatory diseases, or substantial organ damage.

The current methods for reducing uric acid are mainly to reduce production of uric acid and increase excretion of uric acid. Existing drugs for reducing production of uric acid mainly include xanthine oxidase inhibitors such as allopurinol and febuxostat, and drugs that increase excretion of uric acid mainly include renal tubular reabsorption inhibitors such as probenecid, benzbromarone, and Lesinurad.

Although existing methods can effectively reduce the level of uric acid, adverse reactions are worrying. Examples include the hepatic and renal toxicity of allopurinol, the cardiotoxicity of febuxostat, the nephrotoxicity of Lesinurad, etc. In addition, people with high uric acid are mostly middle-aged and elderly people whose organ functions are in decline, and these people usually have relatively poor tolerance to adverse reactions. Therefore, the existing methods for reducing uric acid are greatly limited. There is an urgent need to find a safer method for reducing uric acid.

Lysozyme, also called muramidase, is widely found in nature. It has various pharmacological effects such as antibacterial, antifungal and antitumor effects. It also has the characteristics of good biocompatibility, and no irritation and toxicity to tissues. Thus it has been produced as an enzyme preparation with excellent properties in killing pathogenic microorganisms without damaging the body.

Lysozyme plays an important role in non-specific immunity in the human body. It has a variety of biological activities such as bactericidal and anti-inflammatory effects, promoting tissue repair, and enhancing immunity. It is a pharmaceutical enzyme with excellent properties. Therefore, it has been widely used in the field of medicine, e.g., for the treatment of acute and chronic rhinopharyngitis, oral ulcers, chicken pox, herpes zoster, flat wart, etc.

### SUMMARY

The invention is set out in the appended set of claims.

An object of the present disclosure is to provide lysozyme for use in the prevention or treatment of disease related to an excessively high blood uric acid level.

Another object of the present disclosure is to provide a pharmaceutical composition for preventing or treating a uric acid-related disease, a pharmaceutical composition for reducing uric acid, and a pharmaceutical composition for promoting uric acid excretion.

In particular, the present disclosure relates to the following technical solutions.

Provided is lysozyme for use in the prevention or treatment of a disease related to an excessively high blood uric acid level, wherein the excessively high blood uric acid level is higher than normal blood uric acid level, and the normal blood uric acid level is about less than 420 µmol/L for male and about less than 360 µmol/L for female.

In some embodiments, the disease related to an excessively high blood uric acid level is at least one selected from the group consisting of hyperuricemia, uric acid nephropathy, and gout.

In some embodiments, the disease related to an excessively high blood uric acid level is also accompanied by impaired renal function.

Provided is the use of lysozyme in the preparation of a medicament for reducing uric acid.

In some embodiments, the uric acid is uric acid in the blood of a mammal.

In some embodiments, the mammal is a human being.

In some embodiments, renal function of the mammal is impaired.

Provided is the use of lysozyme in the preparation of a medicament for promoting uric acid excretion.

In some embodiments, the uric acid excretion refers to the excretion of uric acid via the kidneys and/or the intestinal tract.

In some embodiments, the uric acid is uric acid in the blood of a mammal.

In some embodiments, the mammal is a human being.

In some embodiments, renal function of the mammal is impaired.

Provided is a pharmaceutical composition for treating or preventing a uric acid-related disease, wherein the pharmaceutical composition contains lysozyme.

In some embodiments, the uric acid-related disease is at least one selected from the group consisting of hyperuricemia, uric acid nephropathy, and gout.

Provided is a pharmaceutical composition for reducing uric acid, wherein the pharmaceutical composition contains lysozyme.

In some embodiments, the uric acid is uric acid in the blood of a mammal.

In some embodiments, the mammal is a human being.

In some embodiments, renal function of the mammal is impaired.

Provided is a pharmaceutical composition for promoting uric acid excretion, wherein the pharmaceutical composition contains lysozyme.

In some embodiments, the uric acid excretion refers to the excretion of uric acid via the kidneys and/or the intestinal tract.

In some embodiments, the uric acid is uric acid in the blood of a mammal.

In some embodiments, the mammal is a human being.

In some embodiments, renal function of the mammal is impaired.

In some embodiments of the above-mentioned three pharmaceutical compositions, the dosage form of the pharmaceutical composition is any one of an oral preparation, an injection preparation, and an inhalation preparation.

In some embodiments" the oral preparation is an oral enteric preparation, including but not limited to enteric tablets, enteric capsules, enteric soft capsules, enteric pills, enteric pellets, enteric dripping pills, enteric granules, enteric nanoparticles, enteric sustained-release preparations, and enteric controlled-release preparations.

In some embodiments, the oral enteric preparation is a preparation that releases lysozyme in the large intestine, particularly a preparation that releases lysozyme in the colon.

In some embodiments, the oral enteric preparation is a preparation that releases lysozyme in the small intestine, particularly a preparation that releases lysozyme in the ileum or jejunum.

In some embodiments, the potency of the lysozyme in the pharmaceutical composition is more than 20000 U/mg.

For preparing the above-mentioned pharmaceutical compositions, lysozyme can be used alone, or lysozyme can be prepared into a specific preparation form with a pharmaceutically applicable excipient. The pharmaceutically applicable excipient include, but not limited to, a diluent, a filler, a lubricant, a disintegrant, a glidant, an absorption enhancer, a flavoring agent, a polymeric sustained-release material, an enteric matrix material, a coating material, etc.

In some embodiments, the uric acid-related disease refers to a disease related to an abnormal uric acid value in an organism.

In some embodiments, the uric acid-related disease is at least one selected from the group consisting of hyperuricemia, uric acid nephropathy, and gout.

In some embodiments, renal function of the subject suffering from the uric acid-related disease is normal.

In some embodiments, renal function of the subject suffering from the uric acid-related disease is impaired.

In some embodiments, the lysozyme is administered through any dosage form of an oral preparation, an injection preparation, and an inhalation preparation.

In some embodiments, the oral preparation is an oral enteric preparation, including but not limited to enteric tablets, enteric capsules, enteric soft capsules, enteric pills, enteric pellets, enteric dripping pills, enteric granules, enteric nanoparticles, enteric sustained-release preparations, and enteric controlled-release preparations.

In some embodiments, the oral enteric preparation is a preparation that releases lysozyme in the large intestine, particularly a preparation that releases lysozyme in the colon.

In some embodiments, the oral enteric preparation is a preparation that releases lysozyme in the small intestine, particularly a preparation that releases lysozyme in the ileum or jejunum.

In some embodiments, the potency of the lysozyme in the oral preparation is more than 20000 U/mg.

In some embodiments, the dosage of the oral preparation is 0.1-20 g/day.

In some embodiments, the dosage of the oral preparation is 0.3-15 g/day.

In some embodiments, the dosage of the oral preparation is 0.5-6 g/day.

In some embodiments, after the dosage of the oral preparation is determined, the oral preparation is administered 1-3 times a day.

In some embodiments, the uric acid is uric acid in the blood of a mammal.

In some embodiments, the mammal is a human being.

In some embodiments, the blood uric acid level of the subject in need thereof is excessively high.

In some embodiments, renal function of the subject in need thereof is normal.

In some embodiments, in addition to the excessively high blood uric acid level, renal function of the subject in need thereof is impaired.

In some embodiments, the lysozyme is administered through any dosage form of an oral preparation, an injection preparation, and an inhalation preparation.

In some embodiments, the oral preparation is an oral enteric preparation, including but not limited to enteric tablets, enteric capsules, enteric soft capsules, enteric pills, enteric pellets, enteric dripping pills, enteric granules, enteric nanoparticles, enteric sustained-release preparations, and enteric controlled-release preparations.

In some embodiments, the oral enteric preparation is a preparation that releases lysozyme in the large intestine, particularly a preparation that releases lysozyme in the colon.

In some embodiments, the oral enteric preparation is a preparation that releases lysozyme in the small intestine, particularly a preparation that releases lysozyme in the ileum or jejunum.

In some embodiments, the potency of the lysozyme in the oral preparation is more than 20000 U/mg.

In some embodiments, the dosage of the oral preparation is 0.1-20 g/day.

In some embodiments, the dosage of the oral preparation is 0.3-15 g/day.

In some embodiments, the dosage of the oral preparation is 0.5-6 g/day.

In some embodiments, after the dosage of the oral preparation is determined, the oral preparation is administered 1-3 times a day.

In some embodiments, the uric acid is uric acid in the blood of a mammal.

In some embodiments, the mammal is a human being.

In some embodiments, the blood uric acid level of the subject in need thereof is excessively high.

In some embodiments, renal function of the subject in need thereof is normal.

In some embodiments, in addition to the excessively high blood uric acid level, renal function of the subject in need thereof is impaired.

In some embodiments, the lysozyme is administered through any dosage form of an oral preparation, an injection preparation, and an inhalation preparation.

In some embodiments, the oral preparation is an oral enteric preparation, including but not limited to enteric tablets, enteric capsules, enteric soft capsules, enteric pills, enteric pellets, enteric dripping pills, enteric granules, enteric nanoparticles, enteric sustained-release preparations, and enteric controlled-release preparations.

In some embodiments, the oral enteric preparation is a preparation that releases lysozyme in the large intestine, particularly a preparation that releases lysozyme in the colon.

In some embodiments, the oral enteric preparation is a preparation that releases lysozyme in the small intestine, particularly a preparation that releases lysozyme in the ileum or jejunum.

In some embodiments, the potency of the lysozyme in the oral preparation is more than 20000 U/mg.

In some embodiments, the dosage of the oral preparation is 0.1-20 g/day.

In some embodiments, the dosage of the oral preparation is 0.3-15 g/day.

In some embodiments, the dosage of the oral preparation is 0.5-6 g/day.

In some embodiments, after the dosage of the oral preparation is determined, the oral preparation is administered 1-3 times a day.

Beneficial Effects of the disclosure:
The medicaments and uses provided by the present disclosure can significantly reduce the blood uric acid level and have high safety. In particular, for people with relatively poor hepatic and renal function, or people with relatively poor tolerance such as infants, pregnant women, and middle-aged and elderly people, the present disclosure has obvious clinical advantages.

### DETAILED DESCRIPTION

Examples are further listed below to illustrate the present disclosure in detail. It should also be understood that the following examples are only used to further illustrate the present disclosure and should not be construed as limiting the scope of the present disclosure, and some non-essential improvements and adjustments made by those skilled in the art based on the principles set forth in the present disclosure all fall within the scope of the present disclosure. The specific process parameters, etc. in the following examples are also only examples in the appropriate ranges, that is, those skilled in the art would have been able to make selections within the appropriate ranges through the description herein, without limitation to the specific data exemplified hereinbelow.

The references to the methods of treatment by therapy or surgery or *in vivo* diagnosis methods in examples below of this description are to be interpreted as references to compounds, pharmaceutical compositions and medicaments of the present invention for use in those methods.

### Definition:

The Chinese term Rongjunmei, i.e., lysozyme of the present disclosure, can be lysozyme derived from animals, plants or microorganisms, or a recombinant product of natural lysozyme. For example, it can be egg white lysozyme, human lysozyme, recombinant human lysozyme, phage lysozyme, etc. The lysozyme in the present disclosure includes pharmaceutically acceptable salts thereof, such as hydrochloride, chloride, sulfate, or amino acid salts. Lysozyme was first discovered by Fleming as an endogenous enzyme widely present in organisms. Lysozyme has been approved for edible or medicinal use worldwide. In the United States lysozyme has been Generally Recognized as Safe(GRAS). WHO, many European countries, Japan and China have approved it to be used as a food additive. It has been approved for medicinal use in China, Japan, Singapore, and other countries for the treatment of rhinitis, laryngopharyngitis, oral ulcers, chickenpox, herpes zoster, flat warts, etc. Products on the market include Neuzym^{®}, Mucozome^{®}, Laisuorui^{®}, etc. The mechanism of the antibacterial effect of lysozyme mainly involves hydrolyzing peptidoglycan in the cell wall of bacteria, and the mechanism of the antiviral effect mainly involves charge interaction with negatively charged viruses.

Enteric preparation: An enteric preparation refers to a preparation that does not release or hardly releases the drug in the stomach but can release most or all of the drug in certain parts of the intestine when entering the intestine. The human intestine includes the small intestine and the large intestine, wherein the small intestine is further divided into duodenum, jejunum, and ileum, and the large intestine is further divided into cecum, colon, and rectum. Different parts of the digestive tract have different pH values. For example, the pH value in the stomach is about 1-3, the pH value in the small intestine is about 4-7, and the pH value in the large intestine is about 7-8. By selecting a pH-dependent degradable material as an excipient, it is possible to prepare a preparation that directionally releases the drug in a specific part of the digestive tract, e.g., a small intestine enteric preparation or a large intestine enteric preparation. In particular, the preparation includes duodenal enteric preparations, jejunal enteric preparations, ileal enteric preparations, cecal enteric preparations, colonic enteric preparations, or rectal enteric preparations, etc.

uric acid-related disease: A uric acid-related disease refers to a disease related to an abnormal uric acid level in an organism, especially a disease related to an excessively high blood uric acid level, including but not limited to hyperuricemia, gout, uric acid nephropathy, and other diseases caused by high uric acid. The normal blood uric acid level is about less than 420 µmol/L for male and about less than 360 µmol/L for female. When the blood uric acid level is higher than the normal range, it is namely a high uric acid state, which can cause a pathological condition or cause a uric acid-related disease.

Impaired renal function: Impaired renal function refers to abnormal renal function. Clinically, renal function can be evaluated by methods such as determining the serum creatinine level, which is well known to those in the art.

The present disclosure will be specifically illustrated below through animal tests.

Note: The test animals, reagents etc. used in the examples were all commercially available. The relevant test methods used in the experiments, the specific operation methods of the relevant instruments, etc., were well known to those skilled in the art.

Lysozyme granules and enteric lysozyme granules were homemade. The preparation method involved: taking lysozyme with a potency of more than 20000 U/mg as a raw material, adding the same amount of starch, mixing them until uniform, adding a binder (water) for granulation, and drying the granules to obtain lysozyme granules. The prepared lysozyme granules were coated with an enteric coating (HPMC) to obtain enteric lysozyme granules. Benzbromarone fine granules were obtained by grounding commercially available benzbromarone tablets into fine granules.

### Example 1 Effect of lysozyme on the uric acid level of hyperuricemia model animals

### 1.1 Test drug:

Lysozyme granules, enteric lysozyme granules, and benzbromarone fine granules. Each test drug was moistened and uniformly mixed with 0.5% sodium carboxymethylcellulose for easy administration.

### 1.2 Test animal:

SD rats. The rats had free access to drinking water and food, and they were adaptively reared for one week before test.

### 1.3 Grouping and administration:

Except for rats in the blank group, the other rats were modeled for 12 days, and the serum uric acid level of each rat was detected. Then the rats were evenly divided into groups according to the uric acid level of each rat, with 8 animals in each group.

Blank group: No drugs were given.

Model control group: 5 ml of 0.5% sodium carboxymethylcellulose was taken orally once a day.

Lysozyme groups and enteric lysozyme groups: High and low doses of the lysozyme granules and enteric lysozyme granules were respectively taken orally, and the dosages were 10 mg/kg and 30 mg/kg based on the active ingredient, once a day.

Benzbromarone group: The benzbromarone fine granules were taken orally at a dose of 30 mg/kg based on the active ingredient, once a day.

### 1.4 Modeling and test method:

The test was carried out for a total of 18 days.

On day 1 of the test, except for the animals in the blank group, the animals in the other groups were orally given a modeling agent (1.5 g/kg potassium oxonate + 0.3 g/kg uric acid, dissolved in 0.5% sodium carboxymethylcellulose) once to start modeling, and thereafter, the same amount of the modeling agent was administrated at the same time every day.

0.5 ml of blood was taken from the orbit before the start of the test (D0) and on day 12 (D12) of the test, respectively, and centrifuged, then the serum was taken and tested for serum uric acid level using a uric acid assay kit and a microplate reader; in addition, 0.5 g of fresh midsection rat feces were taken, diluted and uniformly mixed with four times volume of phosphate buffer, centrifuged, ultrasonically mixed until uniform, incubated at 100°C for 40 minutes, cooled and then centrifuged, and the supernatant was taken and tested for fecal uric acid level using the uric acid assay kit and the microplate reader.

A significant increase in the serum uric acid levels in the animals indicated that the modeling was successful. Thereafter, the rats were grouped according to the serum uric acid value of each rat. From day 13 (D13), each rat was given the test drug intragastrically every day, as well as the modeling agent every day. The interval between the administration of the test drug and the administration of the modeling agent was more than 1 hour. The drug administration was stopped after day 18 (D18) of the test, and the serum uric acid levels and fecal uric acid levels were determined according to the previous method.

### 1.5 Test results, analysis and processing:

The collected data, statistical analysis, and main results were shown in Tables 1 and 2.

**Table 1: Effects of lysozyme on animal serum uric acid levels (X±SD)**

| Group | Dosage of administration (mg/kg) | Serum uric acid content (µmol/L) | | |
|---|---|---|---|---|
| | | Day 0 | Day 12 | Day 18 |
| Blank group | -- | 41.80±3.63 | 44.18±3.96 | 45.02±4.60 |
| Model control group | -- | 42.26±3.53 | 118.23±13.31^{##} | 192.23±28.67^{##} |
| Benzbromarone group | 30 | 41.02±3.71 | 117.14±17.74^{##} | 153.45±26.17^{##}** |
| Lysozyme group | 10 | 43.66±4.16 | 122.12±10.27^{##} | 161.97±22.11^{##}* |
| | 30 | 40.49±3.33 | 120.79±17.64^{##} | 149.76±20.12^{##}** |
| Enteric lysozyme group | 10 | 44.30±3.09 | 124.71±19.29^{##} | 137.25±24.51^{##}** |
| | 30 | 46.02±3.80 | 118.01±14.80^{##} | 122.38±20.71^{##}** |

| | | | | |
|---|---|---|---|---|
| Note: ## p < 0.01, compared with the blank group; * p < 0.05, compared with the model control group; and ** p < 0.01, compared with the model control group. | | | | |

**Table 2: Effects of lysozyme on animal fecal uric acid levels (X±SD)**

| Group | Dosage of administration (mg/kg) | Fecal uric acid content (mmol/L) | | |
|---|---|---|---|---|
| | | Day 0 | Day 12 | Day 18 |
| Blank group | -- | 1.04±0.17 | 1.02±0.23 | 1.21±0.22 |
| Model control group | -- | 1.13±0.12 | 1.43±0.22^{##} | 1.59±0.19 |
| Benzbromarone group | 30 | 1.12±0.17 | 1.25±0.31 | 1.34±0.31 |
| Lysozyme group | 10 | 0.98±0.13 | 1.26±0.24 | 1.84±0.20^{##} |
| | 30 | 1.15±0.16 | 1.24±0.20 | 1.98±0.20^{##}* |
| Enteric lysozyme group | 10 | 1.07±0.15 | 1.34±0.26^{#} | 2.07±0.31^{##}** |
| | 30 | 1.09±0.12 | 1.29±0.17 | 2.28±0.38^{##}** |

| | | | | |
|---|---|---|---|---|
| Note: # p < 0.05, compared with the blank group; ## p < 0.01, compared with the blank group; * p < 0.05, compared with the model control group; and ** p < 0.01, compared with the model control group. | | | | |

In this test, the method of administering exogenous uric acid and a uricase inhibitor resulted in hyperuricemia in rats. Benzbromarone, a drug that promoted uric acid excretion, was used as a positive control drug. The effects of lysozyme on the uric acid levels of the model animals were observed.

The serum uric acid test results showed that the serum uric acid in the model control group was significantly increased on day 12, indicating that the modeling was successful. On day 18, the serum uric acid levels in the lysozyme group and the enteric lysozyme group were significantly lower than those in the model control group, indicating that lysozyme and enteric lysozyme could both significantly reduce the serum uric acid levels in the model animals. In addition, the effect of the enteric lysozyme in reducing serum uric acid was even superior to that of the positive control drug benzbromarone.

The fecal uric acid test results showed that the fecal uric acid level in the model control group was significantly increased on day 12, indicating that probably due to the stimulating effect of the exogenous uric acid, uric acid excretion via the intestinal tract increased in a compensatory manner. On day 18, the fecal uric acid levels in the lysozyme group and the enteric lysozyme group were significantly higher than those in the model control group, whereas the fecal uric acid levels in the positive drug benzbromarone group did not increase significantly, indicating its incapability to promote uric acid excretion via the intestinal tract; while lysozyme, particularly the enteric lysozyme, could significantly promote uric acid excretion via the intestinal tract.

The promotion of the uric acid excretion via the intestinal tract by lysozyme might be one of the reasons for the reduction of the serum uric acid level. There are two ways to excrete uric acid. Most of uric acid is excreted through the kidney, and a small part of uric acid is excreted through the intestinal tract. URAT1 in the renal tubule is an important transporter for the reabsorption of uric acid into the blood. Inhibition of URAT1 can promote the uric acid excretion through the kidney. Transporters such as ABCG2 in intestinal epithelial cells can transport uric acid in the blood to the intestinal tract and promote the excretion of uric acid through the intestine. At present, an overwhelming majority of drugs which reduce uric acid through promoting the excretion, such as benzbromarone, work by promoting renal excretion. However, for hyperuricemia patients with impaired renal function, the drugs hardly take effect well, in addition they aggravate kidney damage. In contrast, the drugs which promote uric acid excretion via the intestinal tract can reduce the serum uric acid level without being restricted by renal function, protect renal function, and have better safety, therefore presenting obvious advantages.

The administration of lysozyme enteric tablets (produced by Xiangbei Welman Pharmaceutical Co., Ltd.) by two patients with hyperuricemia is described below to specifically illustrate the present disclosure.

### Example 2 Effect of lysozyme enteric tablets on patient with hyperuricemia accompanied by impaired renal function

A 62-year-old female with mild obesity was found to have a serum uric acid value of 550 µmol/L and a serum creatinine value of 192 µmol/L in a physical examination, suggesting moderate hyperuricemia and moderate impaired renal function. Thereafter, she took the lysozyme enteric tablets daily (once in the morning and once in the evening per day, 1 g each time). After two months, a physical examination was performed and it was found that the serum uric acid value decreased to 430 µmol/L, and the serum creatinine value also decreased to 140 µmol/L. After continued use for another two months, the serum uric acid value and the serum creatinine value both returned to normal levels.

### Example 3 Effect of lysozyme enteric tablets on patient with hyperuricemia accompanied by gout

A 51-year-old male suffering from gout had obvious redness, swelling and pain in the ankles and toes, and difficulty in walking. Drugs such as ibuprofen and allopurinol had been used in the past to relieve pain and reduce uric acid; however, the effects were not good, and the serum uric acid value basically remained at a high level of 700 µmol/L or more. The lysozyme enteric tablets were tried daily (once in the morning, once at noon and once in the evening, 2 g each time). One month later, he felt that the frequency of pain episodes decreased and the difficulty in walking was alleviated. After taking **lysozyme enteric tablets** for a total of four months, the serum uric acid value decreased to 580 µmol/L in an examination. After continued use for another three months, the serum uric acid value returned to the normal level, i.e., 400 µmol/L or lower, the redness and swelling were significantly alleviated, and the pain basically no longer occurred.

## Claims

1. Lysozyme for use in the prevention or treatment of disease related to an excessively high blood uric acid level, wherein the excessively high blood uric acid level is higher than normal blood uric acid level, and the normal blood uric acid level is about less than 420 µmol/L for male and about less than 360 µmol/L for female.

2. Lysozyme for use according to claim 1, wherein the disease related to an excessively high blood uric acid level is at least one selected from the group consisting of hyperuricemia, uric acid nephropathy, and gout.

3. Lysozyme for use according to claim 1, wherein renal function of a subject suffering from the disease related to an excessively high blood uric acid level is normal or impaired.

4. Lysozyme for use according to claim 1, wherein the lysozyme prevents or treats the disease related to an excessively high blood uric acid level by reducing uric acid.

5. Lysozyme for use according to claim 1, wherein the lysozyme prevents or treats the disease related to an excessively high blood uric acid level by promoting uric acid excretion.

6. Lysozyme for use according to claim 5, wherein the excretion refers to excretion via the kidney and/or the intestinal tract.

7. Lysozyme for use according to claim 4 or 5, wherein the uric acid is uric acid in the blood of a mammal.

8. Lysozyme for use according to claim 7, wherein the mammal is a human being.

9. Lysozyme for use according to claim 1, 4 or 5, wherein the lysozyme is prepared into any dosage form of an oral preparation, an injection preparation, and an inhalation preparation.

10. Lysozyme for use according to claim 9, wherein the oral preparation is an oral enteric preparation, including but not limited to enteric tablets, enteric capsules, enteric soft capsules, enteric pills, enteric pellets, enteric dripping pills, enteric granules, enteric nanoparticles, enteric sustained-release preparations, and enteric controlled-release preparations.

11. Lysozyme for use according to claim 10, wherein the oral enteric preparation is a preparation that releases lysozyme in the colon, ileum, or jejunum.

12. Lysozyme for use according to claim 9, wherein the potency of lysozyme in the oral preparation is more than 20000 U/mg.

13. Lysozyme for use according to claim 9 or 12, wherein the dosage of lysozyme in the oral preparation is 0.1-20 g/day.

## Patentansprüche

1. Lysozym zur Verwendung bei der Vorbeugung oder Behandlung von Krankheiten, die mit einem übermäßig hohen Blutharnsäurespiegel zusammenhängen, wobei der übermäßig hohe Blutharnsäurespiegel höher ist als der normale Blutharnsäurespiegel und der normale Blutharnsäurespiegel etwa weniger als 420 µmol/L für Männer und etwa weniger als 360 µmol/L für Frauen beträgt.

2. Lysozym zur Verwendung nach Anspruch 1, wobei die Krankheit, die mit einem übermäßig hohen Harnsäurespiegel im Blut zusammenhängt, mindestens eine ist, die aus der Gruppe ausgewählt ist, die aus Hyperurikämie, Harnsäure-Nephropathie und Gicht besteht.

3. Lysozym zur Verwendung nach Anspruch 1, wobei die Nierenfunktion eines Patienten, der an der mit einem übermäßig hohen Harnsäurespiegel im Blut zusammenhängenden Krankheit leidet, normal oder beeinträchtigt ist.

4. Lysozym zur Verwendung nach Anspruch 1, wobei das Lysozym die mit einem übermäßig hohen Harnsäurespiegel im Blut zusammenhängende Krankheit durch Reduzierung der Harnsäure verhindert oder behandelt.

5. Lysozym zur Verwendung nach Anspruch 1, wobei das Lysozym die mit einem übermäßig hohen Harnsäurespiegel im Blut zusammenhängende Krankheit durch Förderung der Harnsäureausscheidung verhindert oder behandelt.

6. Lysozym zur Verwendung nach Anspruch 5, wobei sich die Ausscheidung auf die Ausscheidung über die Niere und/oder den Darmtrakt bezieht.

7. Lysozym zur Verwendung nach Anspruch 4 oder 5, wobei die Harnsäure die Harnsäure im Blut eines Säugetiers ist.

8. Lysozym zur Verwendung nach Anspruch 7, wobei das Säugetier ein Mensch ist.

9. Lysozym zur Verwendung nach Anspruch 1, 4 oder 5, wobei das Lysozym in einer beliebigen Dosierungsform aus einer oralen Zubereitung, einer Injektionszubereitung und einer Inhalationszubereitung hergestellt wird.

10. Lysozym zur Verwendung nach Anspruch 9, wobei das orale Präparat ein orales magensaftresistentes Präparat ist, einschließlich, aber nicht beschränkt auf magensaftresistente Tabletten, magensaftresistente Kapseln, magensaftresistente Weichkapseln, magensaftresistente Pillen, magensaftresistente Pellets, magensaftresistente Tropfpillen, magensaftresistente Granulate, magensaftresistente Nanopartikel, magensaftresistente Präparate mit verzögerter Freisetzung und magensaftresistente Präparate mit kontrollierter Freisetzung.

11. Lysozym zur Verwendung nach Anspruch 10, wobei das orale enterische Präparat ein Präparat ist, das Lysozym im Kolon, Ileum oder Jejunum freisetzt.

12. Lysozym zur Verwendung nach Anspruch 9, wobei der Wirkstoffgehalt von Lysozym in der oralen Zubereitung mehr als 20000 U/mg beträgt.

13. Lysozym zur Verwendung nach Anspruch 9 oder 12, wobei die Dosierung von Lysozym in der oralen Zubereitung 0,1-20 g/Tag beträgt.

## Revendications

1. Lysozyme pour la prévention ou le traitement de maladies liées à un taux d'acide urique sanguin excessivement élevé, dans lequel le taux d'acide urique sanguin excessivement élevé est supérieur au taux d'acide urique sanguin normal, et le taux d'acide urique sanguin normal est inférieur à 420 µmol/L pour les hommes et inférieur à 360 µmol/L pour les femmes.

2. Lysozyme à utiliser selon la revendication 1, dans lequel la maladie liée à un taux d'acide urique sanguin excessivement élevé est au moins une maladie choisie dans le groupe constitué par l'hyperuricémie, la néphropathie à l'acide urique et la goutte.

3. Lysozyme à utiliser selon la revendication 1, dans lequel la fonction rénale d'un sujet souffrant de la maladie liée à un taux d'acide urique sanguin excessivement élevé est normale ou altérée.

4. Lysozyme à utiliser selon la revendication 1, dans lequel le lysozyme prévient ou traite la maladie liée à un taux d'acide urique sanguin excessivement élevé en réduisant l'acide urique.

5. Lysozyme à utiliser selon la revendication 1, dans lequel le lysozyme prévient ou traite la maladie liée à un taux d'acide urique sanguin excessivement élevé en favorisant l'excrétion de l'acide urique.

6. Lysozyme à utiliser selon la revendication 5, dans lequel l'excrétion se réfère à l'excrétion par les reins et/ou le tractus intestinal.

7. Lysozyme à utiliser selon la revendication 4 ou 5, dans lequel l'acide urique est l'acide urique présent dans le sang d'un mammifère.

8. Lysozyme à utiliser selon la revendication 7, dans lequel le mammifère est un être humain.

9. Lysozyme à utiliser selon la revendication 1, 4 ou 5, dans lequel le lysozyme est préparé sous une forme posologique quelconque de préparation orale, de préparation injectable et de préparation pour inhalation.

10. Lysozyme à utiliser selon la revendication 9, dans lequel la préparation orale est une préparation entérique orale, y compris, mais sans s'y limiter, les comprimés entériques, les capsules entériques, les capsules molles entériques, les pilules entériques, les pastilles entériques, les pilules goutte à goutte entériques, les granules entériques, les nanoparticules entériques, les préparations entériques à libération prolongée et les préparations entériques à libération contrôlée.

11. Lysozyme à utiliser selon la revendication 10, dans laquelle la préparation entérique orale est une préparation qui libère le lysozyme dans le côlon, l'iléon ou le jéjunum.

12. Lysozyme à utiliser selon la revendication 9, dans lequel la puissance du lysozyme dans la préparation orale est supérieure à 20000 U/mg.

13. Lysozyme à utiliser selon la revendication 9 ou 12, dans lequel la dose de lysozyme dans la préparation orale est de 0,1 à 20 g/jour.
